# EUROPEAN PATENT APPLICATION

(11) **EP 4 364 740 A1**
(43) Date of publication of application: **08.05.2024**
(21) Application number: 22833558.4
(22) Date of filing: 28.06.2022
(51) Int. Cl.: A61K 31/501, A61K 31/496, A61K 31/4162, A61K 31/7105, A61K 31/713, A61K 45/06, A61P 35/00, G01N 33/68, C12Q 1/6883

(54) **PHARMACEUTICAL COMPOSITION FOR PREVENTING OR TREATING CANCER**

(30) Priority: 28.06.2021 KR 20210083887
(71) Applicant: Industry-Academic Cooperation Foundation Yonsei University, Seoul 120-749 (KR); Veraverse, Seoul 08502 (KR)
(72) Inventor: CHEONG, Jae-Ho, Seoul 06628 (KR); KIM, Jae-woo, Seoul 06574 (KR); FANG, Sungsoon, Yongin-si Gyeonggi-do 16903 (KR); YOON, Bo Kyung, Seoul 04731 (KR); KIM, Hyeonhui, Seoul 04712 (KR)
(74) Representative: V.O.
(86) International application number: PCT/KR2022/009183
(87) International publication number: WO 2023/277502

(57) **Abstract**

The present invention relates to a method for prevention, alleviation, or treatment of cancer patients, especially epithelial mesenchymal transition (EMP)-subtype cancer patients. If PHGDH, SHMT and MTHFD2 inhibitors are co-administered to patients with cancer in which the expression level of a glutaminase (GLS) gene or a protein encoded thereby has increased, 1C metabolism is more effectively inhibited such that there is a synergistic effect on the inhibition of cancer cell proliferation in patients with refractory cancer that is difficult to treat because of recurrence, metastasis, and anticancer drug resistance, thereby enabling cancer to be very effectively treated. In addition, the expression level of the GLS gene or protein encoded thereby is measured so that information related to customized treatment methods from initial stages is provided to each patient and the success of treatment can be increased.

## Description

### FIELD OF THE INVENTION

The present invention relates to a pharmaceutical composition for the prevention or treatment of cancer.

### BACKGROUND TECHNOLOGY

Cancer is one of the incurable diseases that humanity has to solve, and huge amounts of capital are being invested in development to cure it all over the world, and in the case of Korea, it is the number one cause of disease death, with more than 100,000 people diagnosed every year and more than 60,000 deaths.

Carcinogens that cause these cancers include smoking, ultraviolet rays, chemicals, food, and other environmental factors, but it is difficult to develop a treatment due to various causes, and the effects of the treatment vary depending on the site where it occurs. Since the substances currently used as therapeutics are highly toxic and do not selectively eliminate cancer cells, there is an urgent need to develop less toxic and effective anticancer drugs to prevent the occurrence of cancer as well as to treat cancer after it occurs. Despite the rapid advances in cancer diagnosis and treatment over the past decade, the fatality rate from cancer incidence remains high.

ln particular, stomach cancer is one of the most common malignancies and is the third leading cause of cancer mortality worldwide. Despite great progress in the treatment of gastric cancer patients, there are still limitations in the treatment of gastric cancer patients due to frequent recurrence and metastasis, as well as drug resistance.

Research is actively underway on the mechanisms related to cancer recurrence, metastasis, and drug resistance, and among them, the cancer stem cell hypothesis is attracting attention. Studies have shown the presence of stem-like cells, which contribute to tumor aggressiveness, metastasis, recurrence, and resistance to chemotherapy.

### DETAILED DESCRIPTION OF THE INVENTION

### Technical Challenges

The purpose of the present invention is to provide a pharmaceutical composition for the prevention, improvement or treatment of cancer.

Another purpose of the present invention is to provide a method of providing information on treatment options for patients with epithelial mesenchymal transition (EMT) subtype cancer.

However, the technical tasks that the present invention seeks to accomplish are not limited to the tasks mentioned above, and other tasks not mentioned can be clearly understood by persons with ordinary knowledge in the industry from the following descriptions.

### Solving The Challenges

One embodiment of the present invention provides a pharmaceutical composition for the prevention or treatment of cancer.

In an embodiment of the present invention, the pharmaceutical composition comprises a compound capable of inhibiting the expression of a GLS gene, or a pharmaceutically acceptable salt thereof; and an agent capable of inhibiting the expression of any one gene selected from the group consisting of phosphoglycerate dehydrogenase (PHGDH) gene, an Serinehydroxymethyltransferase (SHMT) gene, and an methenyltetrahydrofolatedehydrogenase (NADP + dependent) 2 (MTHFD2 gene), methenyltetrahydrofolatecyclohydrolase (MTHFD gene) or a pharmaceutically acceptable salt thereof as an active ingredient.

In another embodiment of the present invention, the pharmaceutical composition comprises a preparation capable of inhibiting the function of a protein encoded by the GLS gene, or a pharmaceutically acceptable salt thereof; and a preparation capable of inhibiting the function of a protein encoded by any one gene selected from the group consisting of the PHGDH gene, the SHMT gene, and the MTHFD2 gene, or a pharmaceutically acceptable salt thereof as an active ingredient.

The "GLS gene" of the present invention is a gene encoding k-type mitochondrial glutaminase, which catalyzes the hydrolysis of glutamine into glutamate and ammonia. In particular, cancer cells have been reported to overexpress the GLS gene in order to obtain a large amount of energy source and a source necessary for the synthesis of fatty acids, thereby degrading glutamine. For the purposes of the present invention, cancer patients may have overexpression of the GLS gene, which may be characterized by inhibition of proliferation or cell death by agents that can inhibit the expression of the GLS gene or the function of the protein encoded thereby, but is not limited thereto.

The GLS gene of the present invention may consist of a base sequence represented by SEQ ID NO: 1 or SEQ ID NO: 2, but is not limited thereto.

Preparations capable of inhibiting the function of proteins encoded by the GLS gene of the present invention may be compounds labeled with Chemical Formula 1 (CAS No. 1439399-58-2) or Chemical Formula 2 (CAS no. 314045-39-1), but are not limited to:

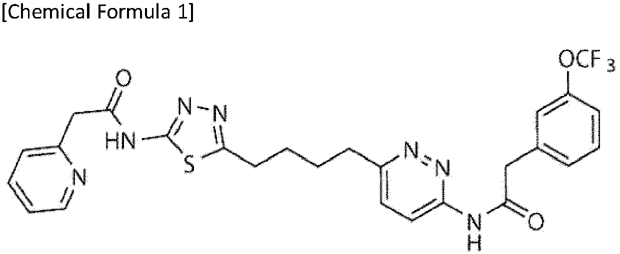

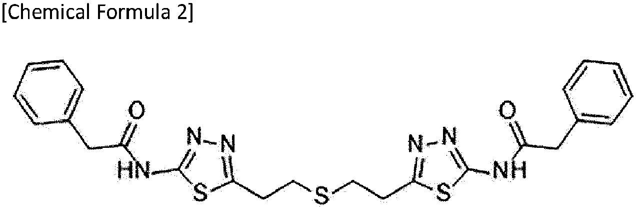

The "PHGDH gene" of the present invention is a gene encoding an enzyme involved in the early stages of L-serine synthesis in animal cells.

The PHGDH gene of the present invention may consist of a base sequence represented by SEQ ID NO: 3, but is not limited thereto.

The "SHMT gene" of the present invention is a gene encoding an enzyme that catalyzes the reversible conversion of serine and tetrahydrofolate to glycine and 5,10-methylentetrahydrofolate.

The SHMT gene of the present invention may consist of a base sequence represented by SEQ ID NO: 4, but is not limited thereto.

The "MTHFD2 gene" of the present invention is a gene encoding an enzyme having methylenetetrahydrofolate dihydrogenase or methenyltetrahydrofolatecyclohydrolase activity.

The MTHFD2 gene of the present invention may consist of a base sequence represented by SEQ ID NO: 5, but is not limited thereto.

Preparations capable of inhibiting the function of proteins encoded by the PHGDH gene of the present invention may be compounds denoted by the following Chemical Formula 3 (CAS No. 1916571-90-8), but are not limited to:

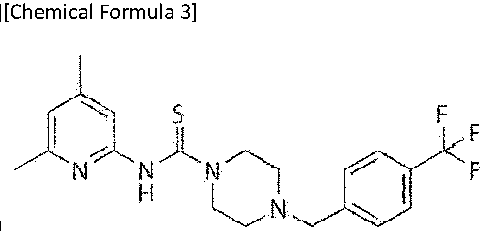

Preparations capable of inhibiting the function of proteins encoded by the MTHFD2 gene of the present invention may be compounds denoted by the following Chemical Formula 4 (CAS No. 2227149-22-4), but are not limited to:

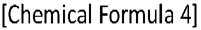

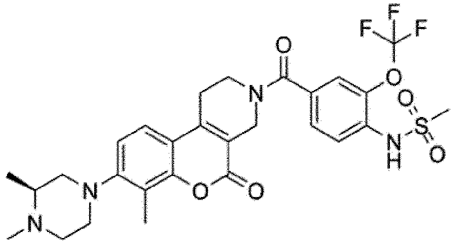

Preparations capable of inhibiting the function of proteins encoded by the SHMT gene of the present invention may be compounds denoted by the following Chemical Formula 5 (CAS No. 2146095-85-2), but are not limited to:

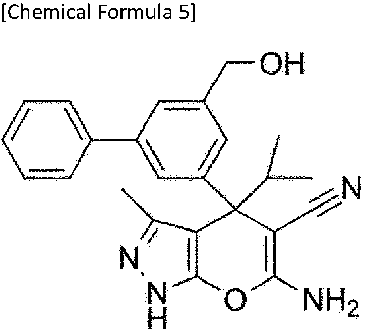

For the purpose of the present invention, the PHGDH, SHMT and MTHFD2 genes and the proteins encoded thereby have increased expression in cancer patients together with the GLS gene and the proteins encoded thereby, so that when administered in combination with a drug that can inhibit the expression of the GLS gene or the function of the protein encoded thereby, it is possible to exert a remarkable synergistic effect on proliferation inhibition and death of cancer cells in cancer patients.

The "pharmaceutically acceptable salt" of the present invention is a salt that is generally considered by a person in the art to be suitable for medical application (e.g., because such salt is not harmful to the subject that can be treated with the salt), or a salt that causes an acceptable side effect within the respective treatment. Generally, the above pharmaceutically acceptable salts are those that are considered acceptable by regulatory authorities such as the U.S. Food and Drug Administration (FDA), the European Medicines Agency (EMA), or the Pharmaceuticals and Medical Devices Agency (PMDA) of the Japanese Ministry of Health and Welfare.

The pharmaceutical composition of the present invention, in each case, the person skilled in the art can easily determine whether a particular compound according to the present invention or a physiologically functional derivative thereof can form a salt, that is, whether the substance corresponding to the inhibitor under the present invention or the physiologically functional derivative thereof, e.g., has an electric charge such as an amino group, a carboxylic acid group, etc.

If the inhibitable Chemical Formulation of the present invention is a compound, the exemplary salt of the compound is an acid adjunct salt or a salt with a base, in particular a pharmaceutically acceptable inorganic and organic acid adjunct salt and a salt with a base commonly used in pharmaceuticals, which is water-insoluble or a particularly water-soluble acid adjunct. Depending on the substituent of the compound, salts with bases may also be suitable. Acid adjunct salt may be formed, for example, by mixing a solution of a compound of the present invention with a solution of a pharmacologically acceptable acid, such as hydrochloric acid, sulfuric acid, fumaric acid, maleic acid, succinic acid, acetic acid, benzoic acid, citric acid, tartaric acid, carbonic acid or phosphoric acid. Likewise, pharmaceutically acceptable base adjunct salts are alkaline metal salts (e.g., sodium or potassium salts); alkaline earth metal salts (for example, calcium or magnesium salts); and salts formed with suitable organic ligands (e.g., ammonium, quaternary ammonium, and amine cations formed using opposite anions such as halides, hydroxides, carboxylates, sulfates, phosphates, nitrates, alkyl sulfonates, and aryl sulfonates). Illustrative examples of pharmaceutically acceptable salts are: acetate, adifate, alginate, arginate, ascorbate, aspartate, benzenesulfonate, benzoate, bicarbonate, bisulfate, biotrate, borate, bromide, butyrate, calcium edetate, camporate, camphosulfonate, camsylate, carbonate, chloride, citrate, digluconate, dihydrochloride, dodecyl sulfate, edetate, edysylate, ethane Sulfonate, Formmate, Fumarate, Galactate, Galacturonate, Gluconate, Glutamate, Glycerophosphate, Hemisulfate, Heptanoate, Hexylesorinate, Hydrobromide, Hydrochloride, Hydroiodide, 2-Hydroxy-Ethane Sulfonate, Hydroxynaphthoate, Iodide, Isobutyrate, Isothionate, Lactate, Laurerate, Lauryl Sulfate, Malate, maleate, malonate, mandelate, methanesulfonate (mesylate), methyl sulfate, 2-naphthalene sulfonate, nicotinate, nitrate, oleate, oxalate, palmitate, pantothenate, pectinate, persulfate, 3-phenylpropionate, phosphate/diphosphate, phthalates, picrate, pibalate, polygalacturonate, propionate, salicylate, stearate, sulfate, suberate, succinate, tannate, tartrate, tosylate, undecanoate, valerate, etc., but is not limited to thereto.

The preparations that can inhibit the expression of the aforementioned genes of the present invention are compounds; It may be, but is not limited to, any one selected from a group of miRNAs, siRNAs, shRNAs, and antisense oligonucleotides that bind specifically to the mRNA of the gene.

The Chemical Formulation capable of inhibiting the function of the protein of the present invention may be one of the compounds, inverse agonists, antagonists, and any one selected from the group consisting of antibodies or aptamers that can bind specifically to the proteins.

The "inverse agonist" or "antagonist" of the present invention means a molecule that can directly or indirectly reduce the biological activity of the receptor, including, but not limited to, a molecule that, when used in conjunction with a ligand of the receptor, may reduce the action of the ligand.

The "antibody" of the present invention means a protein-like molecule which can bind specifically to the antigenic site of a protein or peptide molecule, and such an antibody is obtained by cloning each gene to the expression vector according to the usual method to obtain the protein encoded by the marker gene, and can be prepared by the usual method from the obtained protein.

The "aptamer" of the present invention means a nucleic acid molecule having binding activity to a predetermined target molecule. The aptamer may be RNA, DNA, modified nucleic acids, or a mixture thereof, and may be in the form of a straight chain or a loop, and it is generally known that the shorter the sequence of the nucleotides constituting the aptamer, with easier chemical synthesis and mass production, better cost advantages, cleaner Chemical Formula, better stability in vivo, and lower toxicity.

The above cancers of the present invention include gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin lymphoma, Hodgkin lymphoma, blood cancer, bladder cancer, kidney cancer, melanoma, colon cancer, bone cancer, skin cancer, head cancer, uterine cancer, rectal cancer, brain tumor, cancer, fallopian tube carcinoma, endometrial carcinoma, vaginal cancer, scrotal carcinoma, esophageal cancer, small intestine cancer, endocrine gland cancer, adrenal cancer, soft tissue sarcoma, urethral cancer, penile cancer, ureteral cancer, renal cell carcinoma, renal pelvic carcinoma, CNS central nervous system tumor, It may be any one selected from a group consisting of primary CNS lymphoma, spinal cord tumor, brainstem glioma, and pituitary adenoma, for example, stomach cancer, but not limited to.

The cancer of the present invention may be an incurable cancer, which is a metastasis, recurrence, and drug-resistant cancer, but is not limited to.

The cancer in the present invention may be an epithelial mesenchymal transition (EMT) subtype, but is not limited to.

The "EMT molecular subtype" of the present invention is a subtype in which there is a process of transformation of epithelial cells into mesenchymal cells, and it is a mutation process in which epithelial cells lose their appearance and have the characteristics of mesenchymal cells, and it is known to be an important process in the development of individual formation, and refers to a molecular subtype in which cancer cell growth, drug resistance, infiltration and metastasis may occur.

The composition of the present invention may further include anticancer drugs.

The anticancer drugs of the present invention consist of nitrogen mustard, imatinib, oxaliplatin, rituximab, erlotinib, neratinib, lapatinib, zefitinib, bandantanib, nirotinib, semasanib, bosutinib, axitinib, cediranib, lestaurtinib, trastuzumab, gefitinib, bortezomib, sunitinib, carboplatin, sorafenib, bevacizumab, cisplatin, cetuximab, biscumalbum, asparaginase, tretinoin , hydroxycarbamide, dasatinib, estramostine, gemtuzumapozogamycin, ibritumomab tucetan, heptaplatin, methylaminolevulinic acid, amsacrine, alemtuzumab, procarbazine, alprostadil, holmium nitrate chitosan, gemcitabine, doxyfluridine, pemetrexed, tegapur, capecitabine, zimeracin, oterasil, azacitidine, methotrexate, uracil, cytarabine, Fluorouracil, fludagovine, enositabine, flutamide, kepesitabine, decitabine, mercaptopurine, thioguanine, cladribine, carmofer, ralitrexed, docetaxel, paclitaxel, irinotecan, belotecan, topotecan, vinorellevine, etoposide, vincristine, vinblastine, tenifoside, doxorubicin, idarubicin, epirubicin, mitoxantrone, mitomycin, blelomycin, daunosecinRuvicin, dactinomycin, pyrarubicin, aclarubicin, pepromycin, temsirolimus, temozolomide, busulfan, iphosphamide, cyclophosphamide, melparan, altretmine, dacarbazine, thiotepa, nimustine, chlorambucil, mitolactol, leucovorin, tretonin, exmestane, aminoglutesimide, anagrelide, olaparib, nabelvin, padrazole, tamoxifen, toremifene, testosterone, anastrozole, One or more species selected from the group consisting of letrozole, borozole, bicalutamide, lomustine, borinostat, entinoste, penformin, metformin, talazoparib, and carmustine may be used, but not limited to.

The above "prevention" in the present invention means any act that inhibits or delays the onset of a disease or pathology. For the purposes of the present invention, the composition is meant to delay the onset of cancer, especially EMT molecular subtype cancer, or to inhibit its onset.

The "cure" of the present invention means any act that delays, stops, or reverses the progression of disease or disease, and for the purposes of the present invention the composition means to stop, reduce, mitigate, eliminate or reverse the progression of cancer, especially EMT molecular subtype cancer.

The pharmaceutical composition of the present invention may be characterized as being in the form of capsules, tablets, granules, injections, ointments, powders, or beverages, and the pharmaceutical composition may be characterized by human subjects.

The pharmaceutical composition of the present invention is not limited to these, but each may be Chemical Formulated and used in the form of an oral Chemical Formulation, topical agent, suppository, and sterile injection solution such as an acid, granule, capsule, tablet, aqueous suspension, etc., according to the usual method. The pharmaceutical composition of the present invention may include a pharmacologically acceptable carrier. The pharmacically acceptable carrier can be used as a binder, a synovage, a disintegrating agent, an excipient, a solubilizer, a dispersing agent, a stabilizer, a suspension agent, a color, a fragrance, etc., and in the case of an injection, a buffer, a preservative, a non-monetizing agent, a solubilizing agent, an isotonic agent, a stabilizer, etc., can be used, and in the case of topical administration, a base, excipient, lubricant, preservative, etc. can be used.

The Chemical Formulation of the pharmaceutical composition of the present invention may be prepared in a variety of ways by mixing with a pharmaceutically acceptable carrier as described above. For example, when administered orally, it can be prepared in the form of tablets, troki, capsules, elixirs, suspensions, syrups, wafers, etc., and in the case of injections, it can be prepared in the form of unit-dose ampoules or multi-doses. It can be Chemical Formulated into others, solutions, suspensions, tablets, capsules, extended-release Chemical Formulations, etc.

Examples of carriers, excipients, and diluents suitable for the Chemical Formulation of the present invention include lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate or mineral oil. In addition, it may additionally contain fillers, antiflocculants, lubricants, wetting agents, fragrances, emulsifiers, preservatives, etc.

The route of administration of the pharmaceutical composition of the present invention is not limited to them, but includes oral, intravenous, intramuscular, intraarterial, intramedullary, intrathecal, intracardiac, percutaneous, subcutaneous, intraperitoneal, intranasal, enteral, local, sublingual or rectal. Oral or parenteral dropping is preferable. In the present invention, the "parenteral" includes subcutaneous, intradermal, intravenous, intramuscular, intraarticular, bursal, sternal, intrathecal, intralesional, and intracranial injection or injection techniques. In addition, the pharmaceutical composition can be administered in the form of suppositories for rectal administration.

The pharmaceutical composition of the present invention may vary depending on a number of factors, including the activity of the particular compound used, age, weight, general health, sex, Chemical Formulation, time of administration, route of administration, discharge rate, drug Chemical Formulation, and the severity of the particular disease to be prevented or treated, and the dosage of the pharmaceutical composition depends on the patient's condition, weight, degree of disease, drug form, route and duration of administration, but can be appropriately selected by the person in the art, It can be administered at 0.0001 to 50 mg/kg or 0.001 to 50 mg/kg per day. It can be administered once a day or divided into several doses. The above dosage does not in any way limit the scope of the present invention. The pharmaceutical composition according to the present invention can be Chemical Formulated as a pill, dragee, capsule, liquid, gel, syrup, slurry, suspension.

In another embodiment of the present invention, a Chemical Formulation capable of inhibiting the expression of Glutaminase (GLS) gene in an entity requiring administration, or a pharmaceutically acceptable salt thereof; and a group of Phosphoglycerate Dehydrogenase (PHGDH) genes, Serine hydroxymethyltransferase (SHMT) genes, and Methylenetetrahydrofolate Dehydrogenase (NADP+ Dependent) 2 (MTHFD2) genes, MethenyltetrahydrofolateCyclohydrolase (MTHFD) genes that can inhibit the expression of any one gene, or a drug containing a pharmaceutically acceptable salt thereof in a pharmaceutically effective dose.

In the method of prevention or treatment of the cancer of the present invention, the GLS, PHGDH, SHMT or MTHFD2 genes, preparations capable of inhibiting the expression of the gene, pharmaceutically acceptable salts and cancers are the same as those described in the composition for the prevention, improvement or treatment of the cancer, and are omitted to avoid excessive complexity of the present specification.

In the present invention, the "dosing" means to provide the object with a prescribed composition of the present invention in any appropriate manner.

In the present invention, the "object" requiring the above dosing may include both mammals and non-mammals. Here, examples of said mammals include humans, non-human primates, e.g., chimpanzees, other ape or monkey species; livestock animals, e.g. cattle, horses, sheep, goats, pigs; domesticated animals, e.g. rabbits, dogs or cats; Laboratory animals, such as rodents, e.g. rats, mice or guinea pigs, may include, but are not limited to. In addition, in the present invention, the examples of the non-mammals may include, but are not limited to, birds or fish.

The Chemical Formulation administered as described above in the present invention is not specifically limited, and may be administered as a solid form, liquid form, or aerosol Chemical Formulation for aspiration, and may be administered as a solid form Chemical Formulation intended to be converted into a liquid form Chemical Formulation for oral or parenteral administration immediately prior to use, e.g., an acid, granule, capsule, tablet, oral Chemical Formulation of an aqueous suspension, topical use, suppositories and sterile injection solution, etc. This is not limited to this.

In addition, in the present invention, at the time of the above administration, a pharmacologically acceptable carrier may be additionally administered along with the Chemical Formulation of the present invention. Here, the pharmaceutically acceptable carrier can be used as a binder, synovitizer, disintegrator, excipient, solubilizer, dispersant, stabilizer, suspension agent, color, fragrance, etc., when administered orally, and in the case of injection, a buffer, preservative, non-monetizer, solubilizer, isotonic agent, stabilizer, etc., a mixture can be used, and in the case of topical administration, a base, excipient, lubricant, preservative, etc. can be used. The Chemical Formulation of the compound of the present invention may be prepared in a variety of ways by mixing with a pharmaceutically acceptable carrier as described above. For example, when administered orally, it can be prepared in the form of tablets, trochi, capsules, elixir, suspension, syrup, wafers, etc., and in the case of injections, it can be prepared in the form of unit-dose ampoules or multi-doses. It can be Chemical Formulated as others, solutions, suspensions, tablets, capsules, extended-release Chemical Formulations, etc.

On the other hand, examples of carriers, excipients, and diluents suitable for Chemical Formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate or mineral oil. In addition, it may additionally contain fillers, antiflocculants, lubricants, wetting agents, fragrances, emulsifiers, preservatives, etc.

The route of administration of the preparation pursuant to the present invention is but is not limited to these: oral, intravenous, intramuscular, intra-arterial, intramedullary, intradural, intracardiac, transdermal, subcutaneous, intra-abdominal cavity, intranasal, enteral, topical, sublingual or rectal. Oral or parenteral dropping is preferable.

In the present invention, "parenteral" includes subcutaneous, intradermal, intravenous, intramuscular, intraarticular, bursal, sternal, intrathecal, intralesional, and intracranial injection or injection techniques. The pharmaceutical composition of the present invention can also be administered in the form of a suppository for rectal administration.

In the present invention, "pharmaceutically effective amount" refers to a sufficient amount of an agonist to provide a desired biological outcome. The result may be a reduction and/or alleviation of the signs, symptoms or causes of the disease, or any other desirable change in the biological world. For example, the "effective dose" for therapeutic use is the amount of Chemical Formulation disclosed in the present invention that is required to provide a clinically significant reduction in the disease. The appropriate amount of "effective" in any individual case can be determined by a person skilled in the arts using routine experiments. Thus, the expression "effective dose" usually refers to the amount in which the active substance has a therapeutic effect. In the present case, the active substance is a growth inhibitor of cancer cells and a preventive, ameliorating or curative of cancer.

The Chemical Formulation of the present invention may vary depending on several factors, including activity, age, weight, general health, sex, Chemical Formulation, time of administration, route of administration, discharge rate, drug Chemical Formulation, and severity of the specific disease to be prevented or treated, and the dosage of the preparation varies depending on the patient's condition, weight, degree of disease, drug form, route and duration, but can be appropriately selected by the person in the art, and can be administered at 0.0001 to 100 mg/kg or 0.001 to 100 mg/kg per day. It can be administered once a day or divided into several doses. The above dosage does not in any way limit the scope of the present invention. Compounds according to the present invention may be Chemical Formulated as pills, dragees, capsules, liquids, gels, syrups, slurries, suspensions.

The preparation of the present invention can be used alone or in combination with methods such as surgery, radiotherapy, hormone therapy, chemotherapy, and biologic reaction modulators.

In addition, the Chemical Formulation of the present invention can be used in combination with other anticancer drugs, at which time the anticancer drugs include nitrogen mustard, imatinib, oxaliplatin, rituximab, panitumumab, erlotinib, neratinib, lapatinib, zefitinib, bandantanib, nirotinib, semasanib, bosutinib, axitinib, cediranib, lestaurtinib, trastuzumab, gefitinib, bortezomib, susutricanibNitinib, Carboplatin, 5-Fluorouracil (5-FU), Bevacizumab, Cisplatin, Cetuximab, Applibercept, Regorafenib, Biscumalbum, Asparaginase, Tretinoin, Hydroxycarbamide, Dasatinib, Estramostine, GemtuzumabOzogamycin, Ibritumomab Tucetan, Heptaplatin, Methylaminolevulinic Acid, Amsacrine, Alemtuzumab, Procarbazine, Alprostadil, Holmium Nitrate Chitosan, Gemcitabine, Doxyfluridine, Pemetrexed, Tegapur, Capecitabine, Gimeracin, Oteracil, Azacitidine, Methotrexate, Uracil, Cytarabine, Fluorouracil, FludaGabine, Enositabine, Flutamide, Decitabine, Mercaptopurine, Thioguanine, Cladribine, Leucovorin, Carmoper, Raltitrexed, Interferon alfafa-2a, Docetaxel, paclitaxel, irinotecan, belotecan, topotecan, vinorellevine, etoposide, vincristine, vinblastine, tenifoside, doxorubicin, idarubicin, epirubicin, mitoxantron, mitomycin, blelomycin, daunorubicin, dactinomycin, pyrarubicin, aclavivicin, pepromycin, temsirolimus, temozolomide, 5-fluorouracil, adsulfan, iphosphamide, cyclophosphamide, Melparan, Altretmine, Dacarbazine, Thiotepa, Nimustine, Chlorambucil, Mitolactol, Leucovorin, Trettonin, Exmestane, Aminoglutesimide, Anagrelide, Nabelvin, Padrazole, Tamoxifen, Toremifene, Testosterone, Anastrozole, Letrozole, Borozole, Bicalutamide, Lomustine, and Carmustine may be used., but is not limited to.

Another embodiment of the present invention provides a method of providing information on how to treat patients with EMT molecular subtype cancer.

The method of the present invention is to measure the expression level of the GLS gene or the protein encoded thereby in a biological sample isolated from the object individual; and if the expression level of the gene or protein measured above is increased, the Chemical Formulation that can inhibit the expression of the following genes; or judging by the concomitant administration of a preparation capable of inhibiting the function of proteins encoded by the following genes; Includes:
PHGDH(Phosphoglycerate Dehydrogenase) genes,
SHMT(Serine hydroxymethyltransferase) genes, and
MTHFD2 (Methylenetetrahydrofolate Dehydrogenase (NADP+ Dependent) 2, MethenyltetrahydrofolateCyclohydrolase) genes.

In the present invention, the "object of interest" means a Chemical Formulation capable of inhibiting GLS gene expression; or an individual whose response to treatment is uncertain due to a drug that can inhibit the function of the protein encoded by it, and who has developed cancer or has a high probability of developing cancer.

The "biological sample" of the present invention means any substance, biological body fluid, tissue, or cell obtained from or derived from an individual, e.g., whole blood, leukocytes, peripheral blood mononuclear cells, white blood buffy coat, plasma, and serum) blood, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, It may include, but is not limited to, cells, cell extracts, or cerebrospinal fluid.

The method of measuring the expression level of the protein in the present invention may be at least one selected from the group consisting of protein chip analysis, immunoassay, ligand binding assay, MALDI-TOF (matrix assisted laser description/ionization time of flight mass spectrometry) analysis, SELDI-TOF (surface enhanced laser description/ionization time of flight mass spectrometry) analysis, radiation immuno-diffusion, radiation immunodiffusion, an orotate immune diffusion method, roketimmunoelectrophoresis, tissue immunostaining, complement fixation analysis, two-dimensional electrophoresis analysis, liquid chromatography-mass spectrometry (LC-MS), LC-MS/MS (liquid chromatography/mass spectrometry), western blotting and ELISA (enzyme linked immunosorbent assay).

The expression level of the protein of the present invention can be measured using a Chemical Formulation that can measure the expression level of the protein. A Chemical Formulation capable of measuring the expression level of the protein may be at least one selected from a group consisting of an antibody, an oligopeptide, a ligand, a peptide nucleic acid (PNA) and an aptamer that binds specifically to the protein.

The "antibody" of the present invention refers to a substance that specifically binds to an antigen and produces an antigen-antibody reaction. For the purposes of the present invention, an antibody means an antibody that binds specifically to the protein. The antibodies of the present invention include both polyclonal antibodies, monoclonal antibodies, and recombinant antibodies. The antibody can be readily prepared using a technology widely known in the art industry. For example, polyclonal antibodies may be produced by a method widely known in the art industry, which involves the process of injecting the antigen of the protein into an animal and collecting blood from the animal to obtain a serum containing the antibody. These polyclonal antibodies can be manufactured from any animal, such as goats, rabbits, sheep, monkeys, horses, pigs, cows, and dogs. In addition, monoclonal antibodies are widely known in the industry as the hybridoma method; Kohler and Milstein (1976) European Journal of Immunology 6:511-519), or phage antibody library techniques (Clackson et al, Nature, 352:624-628, 1991; Marks et al, J. Mol. Biol., 222:58, 1-597, 1991). Antibodies prepared by the method can be isolated and purified using methods such as gel electrophoresis, dialysis, salt precipitation, ion exchange chromatography, affinity chromatography, etc. In addition, the antibody of the present invention comprises a complete form having two full-length light chains and two full-length heavy chains, as well as a functional fragment of the antibody.

The functional fragment of the antibody of the present invention means a fragment possessing at least an antigen binding function, and includes Fab, F(ab'), F(ab')2 and Fv.

The "Peptide Nucleic Acid (PNA)" referred to an artificially synthesized polymer similar to DNA or RNA, was first introduced in 1991 by Nielsen, Egholm, Berg and Buchardt of the University of Copenhagen, Denmark. While DNA has a phosphate-ribose saccharide backbone, PNA has a repeated N-(2-aminoethyl)-glycine skeleton linked by peptide binding, which greatly increases its binding strength and stability to DNA or RNA, and is used in molecular biology, diagnostic assays, and antisense therapies.

The "aptamer" of the present invention means an oligonucleic acid or peptide molecule.

Based on the amino acid sequence comprising the proteins of the present invention, the Chemical Formulation corresponding to antibodies, PNAs, and aptamers that bind specifically to the protein can be easily produced by ordinary technicians.

The method of measuring the expression level of the gene of the present invention is to perform reverse transcription polymerase reaction (RT-PCR), competitive reverse transcription polymerase reaction (Competitive RT-PCR), real-time reverse transcription polymerase reaction (Real-time RT-PCR), RNase protection assay (RPA; RNase protection assay), Northern blotting, and at least one selected from a group of DNA chips.

The expression level of the gene of the present invention can be measured using a Chemical Formulation that can measure the expression level of the gene. A Chemical Formulation capable of measuring the expression level of the gene may be at least one selected from a group consisting of primers, probes, and antisense nucleotides that bind complementarily to the gene.

The "primer" of the present invention is a fragment recognizing the target gene sequence, comprising a forward and reverse primer pair, but preferably a primer pair providing assay results of specificity and sensitivity. High specificity can be given when the nucleic acid sequence of the primer is inconsistent with the non-target sequence present in the sample, so that the primer amplifies only the target gene sequence containing a complementary primer binding site and does not induce non-specific amplification.

The "probe" of the present invention means a substance that can bind complementarily with the target substance to be detected in the sample, and means a substance that can specifically confirm the presence of the target substance in the sample through the binding. The type of probe is a material commonly used in the industry, but is not limited to, but may preferably be PNA (peptide nucleic acid), LNA (locked nucleic acid), peptide, polypeptide, protein, RNA, or DNA, and most preferably PNA. More specifically, the probe is a biomaterial that includes biological materials derived from or similar to living organisms or manufactured in vitro, e.g., enzymes, proteins, antibodies, microorganisms, animal and plant cells and organs, neurons, DNA, and RNA, DNA includes cDNA, genomic DNA, oligonucleotides, and RNA includes genomic RNA, mRNA, oligonucleotides.

The "Locked nucleic acids" (LNA) of the present invention means nucleic acid analogues comprising a 2'-O, 4'-C methylene bridge [J Weiler, J Hunziker and J Hall Gene Therapy (2006) 13, 496.502]. LNA nucleosides contain common nucleic acid bases from DNA and RNA, and can form base pairs according to the Watson-Crick base pair rule. However, due to the 'locking' of the molecules caused by the methylene bridge, the LNA is unable to form the ideal shape in the Watson-Crick bond. When LNA is included in DNA or RNA oligonucleotides, it can more quickly pair with complementary nucleotide chains, increasing the stability of the double helix.

The "antisense nucleotide" of the present invention means an oligomer having a nucleotide sequence and an interunit backbone in which the antisense oligomer is hybridized with the target sequence in RNA by Watson-Crick base pair formation, allowing the formation of mRNA and RNA:oligomer hetero dimers, typically within the target sequence. Oligomers can have exact sequence complementarity or approximate complementarity to the target sequence.

Based on the sequence of the genes of the present invention, the Chemical Formulation corresponding to a primer, probe, etc., which binds complementarily to the gene by a conventional engineer based on the sequence of the genes of the present invention, can be easily manufactured.

### Effect of the invention

The present invention relates to a method of prevention, improvement or treatment of cancer patients, especially EMT (epithelial mesenchymal transition) subtype cancer patients, and in cancer patients with increased expression levels of the GLS (glutaminase) gene or the protein encoded thereby, 1C metabolism is more effectively inhibited when administered in combination with PHGDH, SHMT and MTHFD2 inhibitors, and there is a synergistic effect on the inhibition of cancer cell proliferation in patients with incurable cancer that is difficult to treat due to recurrence, metastasis, and anticancer drug resistance.

Furthermore, by measuring the expression level of the GLS gene or the protein encoded by it, it is possible to increase the success of treatment by providing information about personalized treatment options from an early stage to each patient.

### Brief description of the drawing

Figure 1 shows the results of gastric transcriptome analysis in elderly cohort gastric cancer patients according to one embodiment of the present invention.
Figure 2 shows the results of confirming the expression level of proteins encoded by the GLS (glutaminase) gene in intestinal subtype cell lines (NCIN87 and SNU601) and stem-like subtype cell lines (MKN1 and HS746T) according to one embodiment of the present invention through Western blot analysis.
Figure 3 shows the results of genomic analysis performed on intestinal subtype organoids (GA326) and stem-like subtype organoids (GA077) according to one embodiment of the present invention.
Figures 4 to 7 show the results of checking the proliferation level of the cell line according to the presence or absence of glutamine (Fig. 4) and the concentration of DON, which is an analogue of glutamine according to one embodiment of the present invention (Fig. 5), and the result of confirming the proliferation level of the cell line according to the concentration of the GLS inhibitors CB839 (Fig. 6) and BPTES (Fig. 7).
Figure 8 shows the result of confirming the change in the size of the stem-like subform organoid by treatment with the GLS inhibitor CB839 alone according to one embodiment of the present invention.
Figures 9 to 11 show the results of checking the proliferation level of stem-like subtype cell lines with the presence or absence of glutamine and combination treatment with PHGD (Fig. 9), SHMT (Fig. 10) or MTFHD2 (Fig. 11) inhibitors according to one embodiment of the present invention.
Figure 12 shows the result of confirming the change in the size of the stem-like subform organoid by the combination treatment of the GLS inhibitor (CB839) and NCT504 according to one embodiment of the present invention.

### Best Form for the Implementation of the Invention

One embodiment of the present invention includes a Chemical Formulation capable of inhibiting the expression of the glutaminase (GLS) gene, or a pharmaceutically acceptable salt thereof; and a drug that can inhibit the expression of any one gene selected from a group consisting of the PHGDH (Phosphoglycerate Dehydrogenase) gene, the SHMT (Serine hydroxymethyltransferase) gene, and the MTHFD2 gene (Methylenetetrahydrofolate Dehydrogenase (NADP+ Dependent) 2), Methenyltetrahydrofolate Cyclohydrolase, or a pharmaceutical composition for the prevention or treatment of cancer containing a pharmaceutically acceptable salt thereof.

In another embodiment of the present invention, a Chemical Formulation capable of inhibiting the expression of the glutaminase (GLS) gene in an entity requiring administration, or a pharmaceutically acceptable salt thereof; and a method of prevention, improvement or treatment of cancer that includes the administration of a drug that can inhibit the expression of any one gene selected from a group of PHGDH (Phosphoglycerate Dehydrogenase); SHMT (Serine hydroxymethyltransferase) gene, and MTHFD2 (Methylenetetrahydrofolate Dehydrogenase (NADP+ Dependent) 2) gene, MethenyltetrahydrofolateCyclohydrolase, or a composition containing a pharmaceutically acceptable salt thereof as an active ingredient in a pharmacologically effective amount.

Another embodiment of the present invention involves measuring the expression level of the GLS gene or the protein encoded thereby in a biological sample isolated from the object of interest; and if the expression level of the gene or protein measured above is increased, the Chemical Formulation that can inhibit the expression of the following genes; or judging by the concomitant administration of a preparation capable of inhibiting the function of proteins encoded by the following genes; EMT molecules involving subtypes of cancer are related to how to provide information about treatment options.

### EXAMPLES OF THE INVENTION

Hereinafter, the present invention is described in detail by the following embodiment. However, the following embodiment is only an illustration of the present invention, and the content of the present invention is not limited by the following embodiment.

### EXAMPLES

### Example 1: Gastric transcriptome analysis

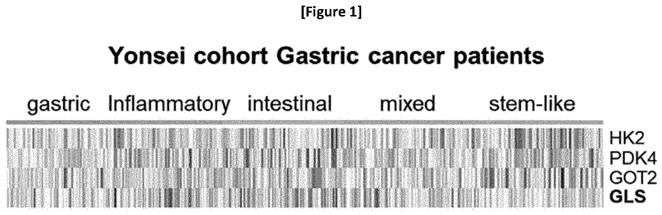

Fresh frozen tumor tissue was obtained from gastric cancer patients who underwent curative intent gastrectomy at Yonsei Cancer Center, and gastric transcriptome analysis data were obtained through the process of matching clinical data. This study was conducted by the Yonsei University College of Medicine Evaluation Board (Institutional Review Board; IRB), and in the case of the above samples, they were collected after written consent from the patient. Gastric cancer patients were divided into 5 subtypes (gastric, inflammatory, intestinal, mixed, and stem-like molecular subtypes) according to the clinically validated classification system, and the genes related to glycolysis and glutaminolysis were analyzed by heatmap according to the usual method, and the results were shown in Figure 1.

As shown in Figure 1, transcriptome analysis in Yonsei cohort gastric patients confirmed an increase in the expression level of GLS (glutaminase) gene in patients classified as stem-like subtypes (EMT molecular subtypes) among gastric cancer subtypes as shown in the heat map.

### Example 2: Confirmation of GLS gene and protein expression levels in gastric cancer cell line

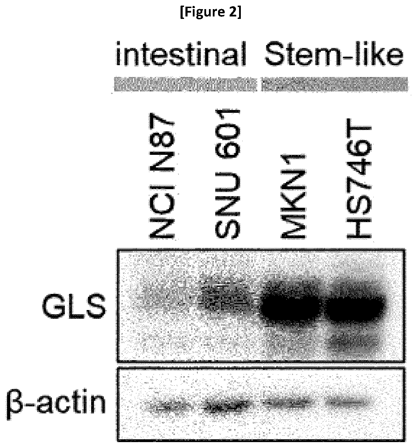

To determine GLS gene and protein expression levels, NCIN87, SNU601, MKN1, and HS746T cell lines were purchased from the Korean cell line bank. Here, in the case of the NCIN87 and SNU601 cell lines, it is a cell line representing the intestinal subtype, and in the case of MKN1 and HS746T cell lines, it corresponds to a cell line representing the stem-like subtype. For these purchased cell lines, RPMI1640 (containing 10% fetal bovine serum (FBS), 2 mM L-glutamine, 100 U/ml penicillin, and 100 µg/ml streptomycin) or DMEM (10% FBS, 2 mM L-glutamine, 100 U/ml penicillin, and 100 µg/ml streptomycin) in a 37 °C, 5% CO₂incubator. At this time, in the case of the above cell lines, all of them were tested for microplasma contamination, and afterconfirming that the mycoplasma was not contaminated, they were used in the experiment. EBC200 (200 mM NaCl, 50 mM Tris-HCl (pH8.0), 0.5% NP-40) containing a protease inhibitor mixture (genedepot) is placed in each cultured cell line. The process of lyssing the cells was carried out. Then, after separating the protein from the lysed cells, the amount of protein was quantified by the BCA analysis method (Pierce), and the same amount of protein was loaded into the SDS-PAGE and electrophoresis was performed, and the SDS-PAGE was transferred to the PVDF membrane (Biorad). After the completed transfer, 5% skim milk (BD difco) was placed in the membrane and blocked for one hour at room temperature, followed by an antibody (abeam) that specifically binds to GLS protein diluted at 1:2000 and β actin diluted at 1:5000, and incubated at 4 °C for one day. Afterwards, add the secondary antibody diluted in 5% skim milk and incubate for 1 hour, and then use LAS 4000 mini (Fujifilm) to check the expression level of the protein. The results are shown in Figure 2.

As shown in Figure 2, compared with NCIN87 and SNU601 cell lines, which correspond to the intestinal subtype, MKN1 and HS746T cell lines, which correspond to the stem-like subtypes, it was found that the expression level of the protein encoded by the GLS gene was significantly increased.

From the above results, it can be seen that the expression level of the protein encoded by the GLS gene is increased, especially in the stem-like subtype compared to other subtypes of gastric cancer.

### Example 3: Genome analysis result of patient-derived organoids

Yonsei University College of Medicine Evaluation Board (Institutional Review Board) IRB) to obtain patient-derived tissues. Then, using the patient-derived tissues, GA326 organoids corresponding to the intestinal subtype and GA077 organoids corresponding to the stem-like subtype were produced.

Specifically, patient tissues classified as enteric or stem-like subtypes according to a clinically validated classification system were obtained and then 40% advanced DMEM/F12 (gibco), 50% Wnt3A cell culture medium (conditioned media), 10% R-spond1 cell culture medium (conditioned media), 1% HEPES (gibco), and 1% GlutaMax (gibco). Organoids were produced using a matrigel to form a 3D structure using a matrigel using a medium containing 0.2% primocin (invivogen), 2% B-27 (invitrogen), 10 mM Nicotinamide (sigma), 1 mM N-Acetylcysteine (sigma), 2 µM A8301, 50 ng/ml mEGF (invitrogen), 100 ng/ml mNoggin (peprotech), 1 nM Gastrin (sigma), 200 ng/ml hFGF10 (peprotech), and 12.5 µM Y-27632 (Enzo).

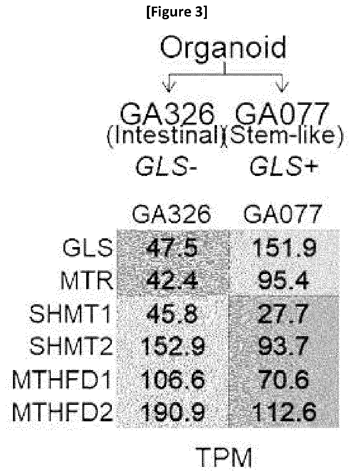

In order to proceed with transcriptome analysis in each of the organoids, the obtained genome analysis data was normalized to TPM, and the transcriptome expression values for the GLS gene and the genes related to monocarbon metabolism, MTR, SHMT1, SHMT2, MTHFD1, and MTHFD2, were identified and shown in Figure 3.

As shown in Figure 3, compared to GA326 organoids corresponding to the intestinal subtype, it was confirmed that the expression level of the GLS gene was significantly increased in the GA077 organoids corresponding to the stem-like subtype.

From the above results, it can be seen that the expression level of the GLS gene is particularly increased in the stem-like subtype compared to other gastric cancer subtypes in organoids with high patient-likeness as well as cell lines.

### Example 4: Confirmation of cell proliferation of stem-like subtype cell lines by GLS inhibitors

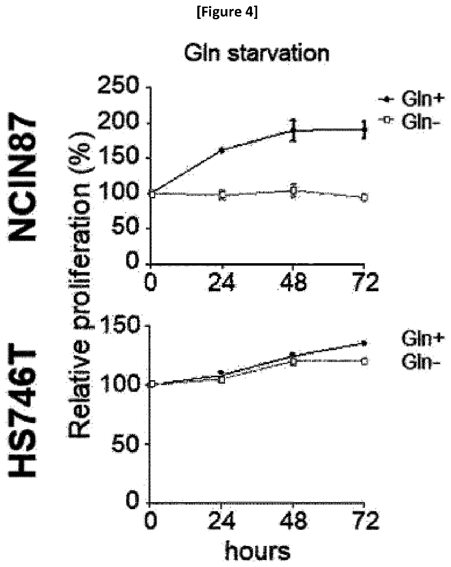

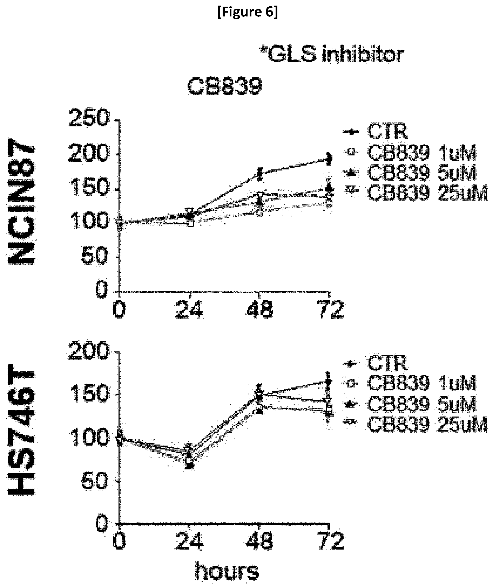

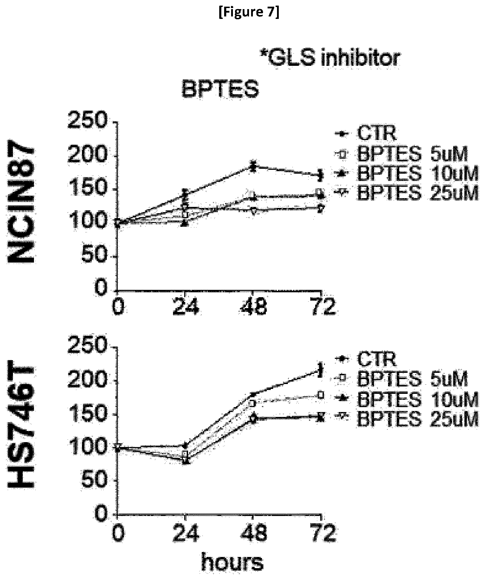

NCIN87, an intestinal subtype cell line, and HS746T, a stem-like subtype cell line, were dispensed in a 96-well plate (black) with 5000 cells each, and replaced with glutamine-deficient culture medium (Gibco) the next day. Then, the cell lines were treated with DON (1 µM, 50 µM, 150 µM; selleckchem, cat no. S8620), CB839 (1 µM, 5 µM, 25 µM; cayman, cat no. S7655) or BPTES (5 µM, 10 µM, 25 µM; MCE, cat no. HY-12683) and the treatment time was 0 hours, and the cell culture medium was discarded at 24, 28 and 72 hours, and stored in an ultra-low temperature freezer at -80°C. After culturing the cells stored in this way at room temperature, 200 µl of cell lysate containing GR dye contained in the cell viability assay kit (CyQUANT cell proliferation assay, Invitrogen) was placed in each well of the cultured cells and incubated at room temperature for 5 min. Afterwards, The intensity of fluorescence was measured at a wavelength of 480 nm and 520 nm excitation using a fluorescence photometer (thermo, varioskan flash 3001), and the measured value was standardized to the number of cells at 0 hour, quantified in %, and the value was shown in Figures 4 to 7.

As shown in Figure 4, cell proliferation was significantly reduced in culture medium (Gln-) without glutamine compared to glutamine-containing culture medium (Gln+) in the intestinal subtype cell line NCIN87, whereas cell proliferation was not reduced with glutamine inclusion in the stem subtype cell line HS746T.

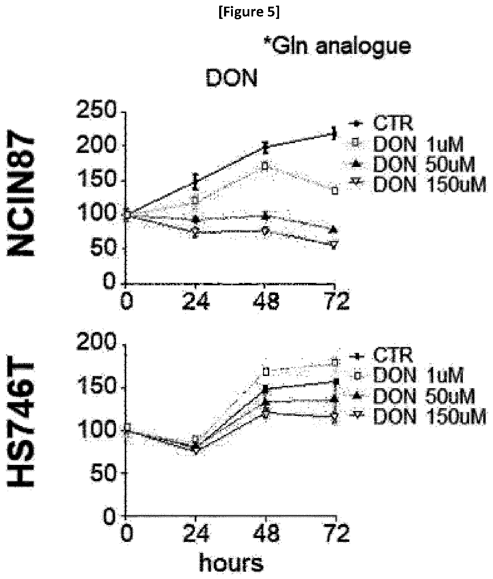

As shown in Figures 5 to 7, cell proliferation was inhibited by glutamine analogues (DON) and GLS activity inhibitors (CB839 and BPTES) in the intestinal subtype cell line NCIN87, whereas cell proliferation was not reduced by the drug in the case of the stem-like cell line HS746T.

From the above results, it can be seen that in the case of stem-like subtypes, that is, refractory cancers, cell proliferation is not inhibited by glutamine deficiency or GLS activity inhibitors.

### Example 5: Confirmation of reduction of proliferation in organoids by GLS inhibitors

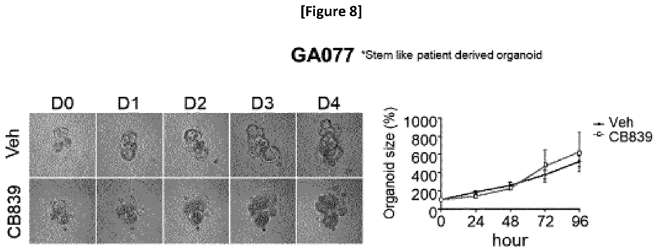

Each of the GA077 organoids produced in Example 3 was treated with 5 µM of CB839 (cayman, cat no. S7655), and the time point of treatment was D0, and it was photographed through an optical microscope (Olympus) on day 1 (D1), day 2 (D2), day 3 (D3), and day 4 (D4), and the longest and shortest diameter passing through the center were measured using image J, and the diameter of the organoid was measured by averaging the two values. Control group (VEH; DMSO-treated group repeated experiment 5 times, and CB839 treatment group repeated experiment 6 times. The organoid diameters measured in this way were converted to µm by microscope size bar, averaged based on the diameter of Day 0, and expressed as a value, which was shown in Figure 8.

As shown in Figure 8, the stem-like subtype GA077 organoid was not reduced in size by CB839, which corresponds to the GLS activity inhibitor.

From the above results, it can be seen that in the case of stem-like subtypes, that is, refractory cancers, cell proliferation is not inhibited by GLS activity inhibitors.

### Example 6: Confirmation of reduction of proliferation in stem-like subtype cell lines by combination therapy

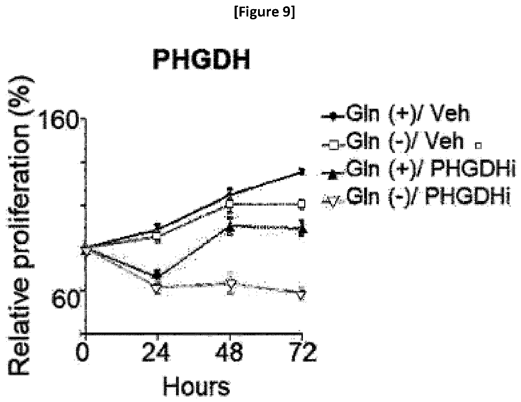

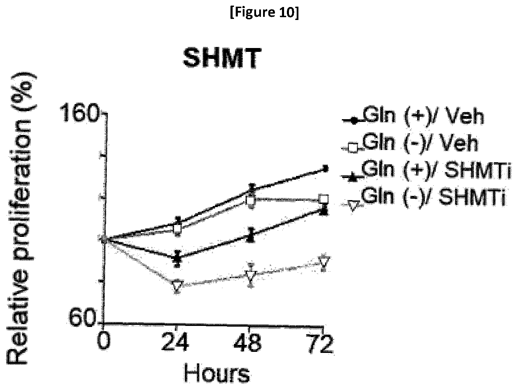

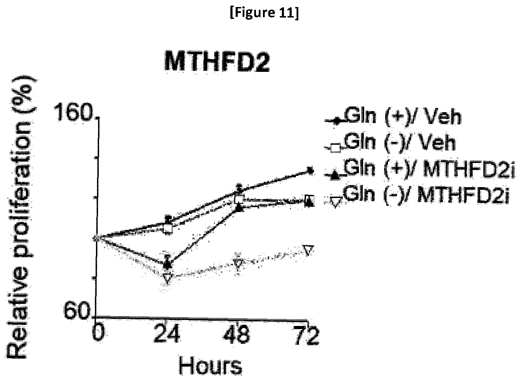

As described in Example 4, after dispensing and incubating the stem-like subtype cell line HS746T, replacing it with a glutamine-deficient culture medium, administering a PHGDH inhibitor (25 µM), SHMT inhibitor (1 µM) or MTHFD2 inhibitor (0.5 µM), and using a cell viability assay kit, the survival rate measured using the cell viability assay kit was shown in Figures 9 to 11.

As shown in Figures 9 to 11, compared to glutamine deficiency alone (Gln(-)/Veh) and each inhibitor alone (Gln(+)/PHGDHi, Gln(+)/SHMTi, Gln(+)/MTHFD2i), glutamine deficiency treated with PHGDH, SHMT, or MTHFD2 inhibitors alone (Gln(-)/PHGDHi, Gln(-)/SHMTi, Gln(-)/MTHFD2i) found a significant synergistic effect on the survival of the stem-like subtype cell line HS746T.

Based on the above results, in the case of glutamine deficiency and stem-like subtypes that are resistant to GLS inhibitors, i.e., refractory cancers, the combination of PHGDH, SHMT, and MTHFD2 inhibitors can effectively inhibit cell survival.

### [Example 7] Confirmation of reduction in proliferation in stem-like subtype organoids by combination therapy

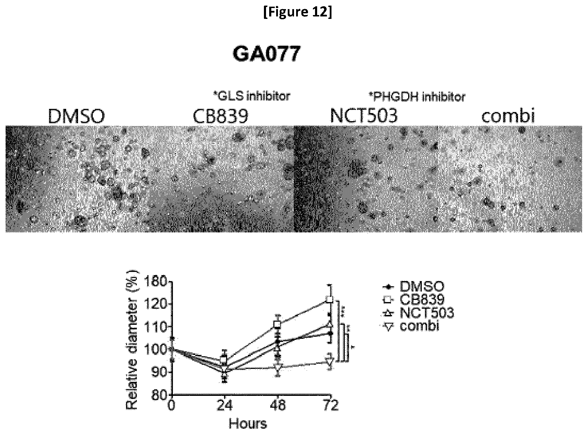

As described in Example 5, GA077, a stem-like subtype organoid, was treated with a GLS inhibitor (CB839, 5 µM), a PHGDH inhibitor (NCT503, 50 µM), or a combination thereof, respectively, and the mean value of the organoid diameter was shown in Figure 12.

As shown in Figure 12, the mean value of organoid diameter was significantly reduced when the stem-like subtype organoid GA077 was treated with either a GLS inhibitor (CB839) or a PHGDH inhibitor (NCT503) alone compared to a combination of them (combi).

The results suggest that in the case of stem-like subtypes that are resistant to glutamine deficiency and GLS inhibitors, the combination of PHGDH, SHMT, and MTHFD2 inhibitors can reduce tumor size very effectively.

The above has been described in detail a specific part of the present invention, and it is clear that for a person with ordinary knowledge of the present industry, this specific description is only a desirable embodiment, and therefore the scope of the present invention is not limited. Accordingly, the substantial scope of the present invention will be defined by the attached claims and their equivalents.

### Industrial Availability

The composition according to the present invention can not only inhibit the proliferation of cancer cells, but also has a synergistic effect on the inhibition of the proliferation of cancer cells in patients with incurable cancer that is difficult to treat due to the presence of recurrence, metastasis, and resistance to anticancer drugs, so that cancer can be treated very effectively.

### SEQUENCE LISTING TEXT

SEQ ID NO: 1. GLS 
atgatgcggctgcgaggctcggggatgctgcgggacctgctcctgcggtcgcccgccggcgtgagcgcgactctgcggcgggcacagcccttg
gtcaccctgtgccggcgtccccgaggcgggggacggccggccgcgggcccggctgccgccgcgcgactccacccgtggtggggcgggggcggctgg
ccggcggagcccctcgcgcggggcctgtccagctctccttcggagatcttgcaggagctgggcaaggggagcacgcatccgcagcccggggtgtcgc
cacccgctgccccggcggcgcccggccccaaggacggccccggggagacggacgcgtttggcaacagcgagggcaaagagctggtggcctcaggt
gaaaataaaataaaacagggtctgttacctagcttggaagatttgctgttctatacaattgctgaaggacaagagaaaatacctgttcataaatttatt
acagcactcaaatctacaggattgcgaacgtctgatcccaggttgaaagagtgtatggatatgttaagattaactcttcaaacaacatcagatggtgtc
atgctagacaaagatctttttaaaaaatgtgttcagagcaacattgttttgttgacacaagcatttagaagaaagtttgtgattcctgactttatgtctttt
acctcacacattgatgagttatatgaaagtgctaaaaagcagtctggaggaaaggttgcagattatattcctcaactggccaaattcagtcccgatttg
tggggtgtgtctgtttgtacagtagatggacagaggcattctactggagataccaaagttcccttctgtcttcagtcctgtgtaaaacctttgaaatatgc
cattgctgttaatgatcttggaactgaatatgtgcatcgatatgttggaaaagagccgagtggactaagattcaacaaactatttttgaatgaagatga
taaaccacataatcctatggtaaatgctggagcaattgttgtgacttcactaataaagcaaggagtaaataatgctgaaaaatttgactatgtcatgca
gtttttgaataagatggctggtaatgaatatgttggattcagtaatgcaacgtttcagtctgaaagagaaagtggagatcgaaattttgcaataggata
ttacttaaaagaaaagaagtgttttccagaaggcacagacatggttggtatattagacttctacttccagctgtgctccattgaagtgacttgtgaatca
gccagtgtgatggctgcgacactggctaatggtggtttctgcccaattactggtgaaagagtactgagccctgaagcagttcgaaatacattgagtttg
atgcattcctgtggcatgtatgacttctcagggcagtttgctttccatgttggtcttcctgcaaaatctggagttgctgggggcattcttttagttgtcccca
atgttatgggtatgatgtgctggtctcctcctctggataagatgggcaacagtgttaagggaattcacttttgtcacgatcttgtttctctgtgtaatttcca
taactatgataatttgagacactttgcaaaaaaacttgatcctcgaagagaaggtggtgatcaaagggtaaagtcagtgataaatcttttgtttgctgc
atatactggagatgtgtctgcacttcgaagatttgctttgtcagctatggacatggaacagcgggactatgattctagaacagcactccatgtagctgct
gcagagggtcatgttgaagttgttaaatttttgctggaagcctgcaaagtaaaccctttccccaaggacaggtggaataacactcccatggatgaagc
actgcactttggacaccatgatgtatttaaaattctccaagaataccaagtccagtacacacctcaaggagattctgacaacgggaaggaaaatcaaa
ccgtccataagaatcttgatggattgttgtaa
[148] SEQ ID NO:2. GLS 
atgatgcggctgcgaggctcggggatgctgcgggacctgctcctgcggtcgcccgccggcgtgagcgcgactctgcggcgggcacagcccttg
gtcaccctgtgccggcgtccccgaggcgggggacggccggccgcgggcccggctgccgccgcgcgactccacccgtggtggggcgggggcggctgg
ccggcggagcccctcgcgcggggcctgtccagctctccttcggagatcttgcaggagctgggcaaggggagcacgcatccgcagcccggggtgtcgc
cacccgctgccccggcggcgcccggccccaaggacggccccggggagacggacgcgtttggcaacagcgagggcaaagagctggtggcctcaggt
gaaaataaaataaaacagggtctgttacctagcttggaagatttgctgttctatacaattgctgaaggacaagagaaaatacctgttcataaatttatt
acagcactcaaatctacaggattgcgaacgtctgatcccaggttgaaagagtgtatggatatgttaagattaactcttcaaacaacatcagatggtgtc
atgctagacaaagatctttttaaaaaatgtgttcagagcaacattgttttgttgacacaagcatttagaagaaagtttgtgattcctgactttatgtctttt
acctcacacattgatgagttatatgaaagtgctaaaaagcagtctggaggaaaggttgcagattatattcctcaactggccaaattcagtcccgatttg
 
tggggtgtgtctgtttgtacagtagatggacagaggcattctactggagataccaaagttcccttctgtcttcagtcctgtgtaaaacctttgaaatatgc
cattgctgttaatgatcttggaactgaatatgtgcatcgatatgttggaaaagagccgagtggactaagattcaacaaactatttttgaatgaagatga
taaaccacataatcctatggtaaatgctggagcaattgttgtgacttcactaataaagcaaggagtaaataatgctgaaaaatttgactatgtcatgca
gtttttgaataagatggctggtaatgaatatgttggattcagtaatgcaacgtttcagtctgaaagagaaagtggagatcgaaattttgcaataggata
ttacttaaaagaaaagaagtgttttccagaaggcacagacatggttggtatattagacttctacttccagctgtgctccattgaagtgacttgtgaatca
gccagtgtgatggctgcgacactggctaatggtggtttctgcccaattactggtgaaagagtactgagccctgaagcagttcgaaatacattgagtttg
atgcattcctgtggcatgtatgacttctcagggcagtttgctttccatgttggtcttcctgcaaaatctggagttgctgggggcattcttttagttgtcccca
atgttatgggtatgatgtgctggtctcctcctctggataagatgggcaacagtgttaagggaattcacttttgtcacgatcttgtttctctgtgtaatttcca
taactatgataatttgagacactttgcaaaaaaacttgatcctcgaagagaaggtggtgatcaaaggcattcctttggaccattggactatgaaagtct
ccaacaagaacttgctttaaaagagacagtatggaaaaaagtgtcacctgagtcaaatgaggacatctctacaactgtagtatatagaatggaaagt
ctgggagagaaaagctaa
[150] SEQ ID NO:3. PHGDH 
atggcttttgcaaatctgcggaaagtgctcatcagtgacagcctggacccttgctgccggaagatcttgcaagatggagggctgcaggtggtg
gaaaagcagaaccttagcaaagaggagctgatagcggagctgcaggactgtgaaggccttattgttcgctctgccaccaaggtgaccgctgatgtca
tcaacgcagctgagaaactccaggtggtgggcagggctggcacaggtgtggacaatgtggatctggaggccgcaacaaggaagggcatcttggttat
gaacacccccaatgggaacagcctcagtgccgcagaactcacttgtggaatgatcatgtgcctggccaggcagattccccaggcgacggcttcgatg
aaggacggcaaatgggagcggaagaagttcatgggaacagagctgaatggaaagaccctgggaattcttggcctgggcaggattgggagagaggt
agctacccggatgcagtcctttgggatgaagactatagggtatgaccccatcatttccccagaggtctcggcctcctttggtgttcagcagctgcccctg
gaggagatctggcctctctgtgatttcatcactgtgcacactcctctcctgccctccacgacaggcttgctgaatgacaacacctttgcccagtgcaaga
agggggtgcgtgtggtgaactgtgcccgtggagggatcgtggacgaaggcgccctgctccgggccctgcagtctggccagtgtgccggggctgcactg
gacgtgtttacggaagagccgccacgggaccgggccttggtggaccatgagaatgtcatcagctgtccccacctgggtgccagcaccaaggaggctc
agagccgctgtggggaggaaattgctgttcagttcgtggacatggtgaaggggaaatctctcacgggggttgtgaatgcccaggcccttaccagtgcc
ttctctccacacaccaagccttggattggtctggcagaagctctggggacactgatgcgagcctgggctgggtcccccaaagggaccatccaggtgat
aacacagggaacatccctgaagaatgctgggaactgcctaagccccgcagtcattgtcggcctcctgaaagaggcttccaagcaggcggatgtgaac
ttggtgaacgctaagctgctggtgaaagaggctggcctcaatgtcaccacctcccacagccctgctgcaccaggggagcaaggcttcggggaatgcct
cctggccgtggccctggcaggcgccccttaccaggctgtgggcttggtccaaggcactacacctgtactgcaggggctcaatggagctgtcttcaggcc
agaagtgcctctccgcagggacctgcccctgctcctattccggactcagacctctgaccctgcaatgctgcctaccatgattggcctcctggcagaggc
aggcgtgcggctgctgtcctaccagacttcactggtgtcagatggggagacctggcacgtcatgggcatctcctccttgctgcccagcctggaagcgtg
gaagcagcatgtgactgaagccttccagttccacttctaa
[152] SEQ ID NO:4. SHMT2 
atgctgtacttctctttgttttgggcggctcggcctctgcagagatgtgggcagctggtcaggatggccattcgggctcagcacagcaacgcagc
ccagactcagactggggaagcaaacaggggctggacaggccaggagagcctgtcggacagtgatcctgagatgtgggagttgctgcagagggaga
aggacaggcagtgtcgtggcctggagctcattgcctcagagaacttctgcagccgagctgcgctggaggccctggggtcctgtctgaacaacaagtac
tcggagggttatcctggcaagagatactatgggggagcagaggtggtggatgaaattgagctgctgtgccagcgccgggccttggaagcctttgacct
ggatcctgcacagtggggagtcaatgtccagccctactccgggtccccagccaacctggccgtctacacagcccttctgcaacctcacgaccggatcat
ggggctggacctgcccgatgggggccatctcacccacggctacatgtctgacgtcaagcggatatcagccacgtccatcttcttcgagtctatgccctat
aagctcaacctggcactgactgctcgacttttccggccacggctcatcatagctggcaccagcgcctatgctcgcctcattgactacgcccgcatgaga
gaggtgtgtgatgaagtcaaagcacacctgctggcagacatggcccacatcagtggcctggtggctgccaaggtgattccctcgcctttcaagcacgc
ggacatcgtcaccaccactactcacaagactcttcgaggggccaggtcagggctcatcttctaccggaaaggggtgaaggctgtggaccccaagact
ggccgggagatcccttacacatttgaggaccgaatcaactttgccgtgttcccatccctgcaggggggcccccacaatcatgccattgctgcagtagct
gtggccctaaagcaggcctgcacccccatgttccgggagtactccctgcaggttctgaagaatgctcgggccatggcagatgccctgctagagcgagg
ctactcactggtatcaggtggtactgacaaccacctggtgctggtggacctgcggcccaagggcctggatggagctcgggctgagcgggtgctagagc
 
ttgtatccatcactgccaacaagaacacctgtcctggagaccgaagtgccatcacaccgggcggcctgcggcttggggccccagccttaacttctcgac
agttccgtgaggatgacttccggagagttgtggactttatagatgaaggggtcaacattggcttagaggtgaagagcaagactgccaagctccaggat
ttcaaatccttcctgcttaaggactcagaaacaagtcagcgtctggccaacctcaggcaacgggtggagcagtttgccagggccttccccatgcctggt
tttgatgagcattga
[154]SEQ ID NO:5. MTHFD2

atggctgcgacttctctaatgtctgctttggctgcccggctgctgcagcccgcgcacagctgctcccttcgccttcgccctttccacctcgcggca
gttcgaaatgaagctgttgtcatttctggaaggaaactggcccagcagatcaagcaggaagtgcggcaggaggtagaagagtgggtggcctcaggc
aacaaacggccacacctgagtgtgatcctggttggcgagaatcctgcaagtcactcctatgtcctcaacaaaaccagggcagctgcagttgtgggaat
caacagtgagacaattatgaaaccagcttcaatttcagaggaagaattgttgaatttaatcaataaactgaataatgatgataatgtagatggcctcct
tgttcagttgcctcttccagagcatattgatgagagaaggatctgcaatgctgtttctccagacaaggatgttgatggctttcatgtaattaatgtaggac
gaatgtgtttggatcagtattccatgttaccggctactccatggggtgtgtgggaaataatcaagcgaactggcattccaaccctagggaagaatgtgg
ttgtggctggaaggtcaaaaaacgttggaatgcccattgcaatgttactgcacacagatggggcgcatgaacgtcccggaggtgatgccactgttaca
atatctcatcgatatactcccaaagagcagttgaagaaacatacaattcttgcagatattgtaatatctgctgcaggtattccaaatctgatcacagca
gatatgatcaaggaaggagcagcagtcattgatgtgggaataaatagagttcacgatcctgtaactgccaaacccaagttggttggagatgtggattt
tgaaggagtcagacaaaaagctgggtatatcactccagttcctggaggtgttggccccatgacagtggcaatgctaatgaagaataccattattgctg
caaaaaaggtgctgaggcttgaagagcgagaagtgctgaagtctaaagagcttggggtagccactaattaa

## Claims

1. A pharmaceutical composition for prevention of treatment of cancer comprising:
a compound or a pharmaceutically acceptable salts thereof for inhibiting expression of a glutaminase (GLS) gene; and
an agent or a pharmaceutically acceptable salt thereof for inhibiting the expression of a gene selected from the group consisting of a Phosphoglycerate Dehydrogenase (PHGDH) gene, a SHMT (Serine hydroxymethyltransferase (SHMT) gene, and an MTHFD2 (Methylenetetrahydrofolate Dehydrogenase (NADP+ Dependent) 2) gene, MethenyltetrahydrofolateCyclohydrolase gene.

2. The pharmaceutical composition of claim 1, wherein the agent specifically binds to the mRNA of the gene and is selected from the group consisting of an miRNA, siRNA, shRNA and antisense oligonucleotide.

3. The pharmaceutical composition of claim 1, further comprising an anticancer agent.

4. The pharmaceutical composition of claim 1, wherein the cancer is an epithelial mesenchymal transition (EMT) subtype.

5. The pharmaceutical composition of claim 1, wherein the cancer is gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin lymphoma, Hodgkin's lymphoma, blood cancer, bladder cancer, head cancer, uterine cancer, rectal cancer, brain tumor, cancer, esophageal cancer, small intestinal cancer, endocrine adenocarcinoma, renal cancer, soft tissue sarcoma, urethral cancer, penis cancer, The pharmaceutical composition of any one selected from the group consisting of renal cell carcinoma, renal pelvic carcinoma, central nerve system (CNS) tumor, primary CNS lymphoma, spinal cord tumor, brain glioma, and pituitary adenoma.

6. A pharmaceutical composition for prevention of treatment of cancer comprising:
a compound or a pharmaceutically acceptable salts thereof for inhibiting a function of a protein expressed by glutaminase (GLS) gene; and
an agent or a pharmaceutically acceptable salt thereof for inhibiting a function of a protein expressed by a gene selected from the group consisting of a PHGDH gene, an SHMT gene, and an MTHFD2 (Methylenetetrahydrofolate Dehydrogenase (NADP+ Dependent) 2) gene, MethenyltetrahydrofolateCyclohydrolase gene.

7. The pharmaceutical composition of claim 6, wherein the compound of the agent is an inverse agonist, or an antagonist, antibody or an aptamer binding to the protein selectively.

8. The pharmaceutical composition of claim 6, further comprising an anticancer agent.

9. The pharmaceutical composition of claim 6, wherein the cancer is an epithelial mesenchymal transition (EMT) subtype.

10. The pharmaceutical composition of claim 6, wherein the cancer is gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin lymphoma, Hodgkin's lymphoma, blood cancer, bladder cancer, head cancer, uterine cancer, rectal cancer, brain tumor, cancer, esophageal cancer, small intestinal cancer, endocrine adenocarcinoma, renal cancer, soft tissue sarcoma, urethral cancer, penis cancer, The pharmaceutical composition of any one selected from the group consisting of renal cell carcinoma, renal pelvic carcinoma, central nerve system (CNS) tumor, primary CNS lymphoma, spinal cord tumor, brain glioma, and pituitary adenoma.

11. A method for providing information for treating a patient with an epithelial mesenchymal transition (EMT) subtype comprising:
measuring an expression levels of a GLS gene or a protein encoded by it in a biological sample isolated from the object of interest; and
if the expression level of the gene or protein measured above is elevated, determining to concomitantly administrate a composition for inhibiting expression of the following genes and/or an agent capable of inhibiting function of the protein.
